# EUROPEAN PATENT SPECIFICATION

(11) **EP 3 999 069 B1**
(45) Date of publication and mention of the grant of the patent: **11.10.2023**
(21) Application number: 20740022.7
(22) Date of filing: 17.07.2020
(51) Int. Cl.: A61K 31/496, A61K 38/21, A61P 31/20, A61K 31/52

(54) **SYNERGISTIC EFFECT OF EYP001 AND PEGYLATED IFN-ALPHA FOR THE TREATMENT OF HBV INFECTION**
SYNERGISTISCHE WIRKUNG VON EYP001 UND PEGYLATED IFN-ALPHA ZUR BEHANDLUNG EINER HBV-INFEKTION
EFFET SYNERGIQUE DE AGONISTE EYP001 ET IFN-ALPHA PEGYLE POUR LE TRAITEMENT DE L'INFECTION DU VIRUS DE L'HÉPATITE B (HBV)

(30) Priority: 18.07.2019 EP 19186941
(43) Date of publication of application: 25.05.2022
(73) Proprietor: ENYO Pharma, 69008 Lyon (FR)
(72) Inventor: VONDERSCHER, Jacky, 81600 SENOUILLAC (FR); ROY, Elise, 1012 LAUSANNE (CH); DARTEIL, Raphaël, 69006 LYON (FR); SCALFARO, Pietro, 3984 FIESCH (CH)
(74) Representative: Cabinet Becker et Associés
(86) International application number: PCT/EP2020/070241
(87) International publication number: WO 2021/009333

(56) References cited:
- EP-A1- 1 886 685
- WO-A1-2015/036442
- CN-A- 107 441 098
- ERKEN R ET AL: "FRI-286: First clinical evaluation in chronic hepatitis B patients of the synthetic farnesoid X receptor agonist EYP001", JOURNAL OF HEPATOLOGY; 53RD ANNUAL MEETING OF THE EUROPEAN ASSOCIATION FOR THE STUDY OF THE LIVER, INTERNATIONAL LIVER CONGRESS 2018, ELSEVIER BV, DK; PARIS, FRANCE, vol. 68, no. Supplement 1, 1 April 2018 (2018-04-01), pages S488-S489, XP009518138, ISSN: 1600-0641, DOI: 10.1016/S0168-8278(18)31226-1 [retrieved on 2018-04-23]
- JOLY ET AL.: "The selective FXR agonist EYP001 is well tolerated in healthy subjects and has additive anti-HBV effect with nucleoside analogues in HepaRG cells", JOURNAL OF HEPATOLOGY 20180401 ELSEVIER B.V. NLD, vol. 68, no. Supplement 1, 1 April 2018 (2018-04-01), XP085012937, ISSN: 1600-0641

## Description

### FIELD OF THE INVENTION

The present invention relates to a method for treating Hepatitis B infection.

### BACKGROUND OF THE INVENTION

Hepatitis B remains a major worldwide public health problem with over 350 million of chronically-infected people despite extensive vaccination programs. Chronic hepatitis B evolves towards life threatening complications including liver cirrhosis and cancer. Current therapeutic regimens are long term treatments (e.g., polymerase inhibitors, life long; pegylated interferons up to one year) and fail to cure HBV as they do not target the virus reservoir. HBV functional cure remains a major unmet medical need.

The primary goal of treatment for chronic hepatitis B (CHB) is to permanently suppress HBV replication and prevent or improve liver disease. Seven drugs are currently available for treatment of CHB infection - conventional interferon (IFN), pegylated interferon and direct antiviral agents. The direct antivirals (nucleos/tide analogues) belong to three classes: L-nucleosides (lamivudine, telbivudine and emtricitabine); deoxyguanosine analogs (entecavir) and nucleoside phosphonates (adefovir and tenofovir) which directly interfere with HBV DNA replication, primarily as chain terminators. The key limitations for interferon treatment are major side- effects, low rate of HBV DNA suppression and low rate of ALT normalization; key limitations of the treatment with direct antivirals are: development of resistance; rebound of HBV replication after stopping therapy requiring prolonged, life-long therapy, very low rate of HBsAg clearance, increasing the risk of adverse events with prolonged, life-long therapy. Importantly, current direct antivirals repress the reverse transcription of the pre-genomic viral RNA into the genomic DNA. They thus act downstream to the formation of the covalently closed circular DNA (cccDNA) that is formed after virus entry into hepatocytes. cccDNA reside in the cell nucleus as additional minichromosomes that are transcribed into viral mRNAs and transmitted to daughter cells when hepatocytes divide. Current direct antivirals have no or very little effect on the HBV cccDNA reservoir and the expression of the viral genes. Thus, the currently available treatments are suboptimal and may be associated with severe side effects.

WO 2015/036442 discloses the interest of FXR agonist for decreasing HBV replication. EYP001 is a synthetic non-steroidal, non-bile acid FXR agonist with a good tolerability profile. EYP001 is an orally bioavailable small molecule currently evaluated in phase Ib in patients with chronic hepatitis B. Contrary to lifelong standards of care that target essentially virus replication, EYP001 is targeting the cccDNA ('virus reservoir'), therefore aiming for HBV real cure. Erken et al (2018, Journal of Hepatology, 68, Suppl 1, S488-S489) discloses that EYP001 reduces HBV viral load in chronic hepatitis B patients. Joly et al (2017, Journal of Hepatology, 66, Suppl 1, SAT-158) discloses that EYP001 and nucleosides analogue can be safely used in healthy individuals and have additive effects on HBV reduction/elimination in cell culture.

Several combined therapies with IFN and nucleo(s/t)ide analogs have been studied: namely IFN-α and a drug selected from lamivudine, adefovir, telbuvirine, entecavir and tenofovir (Woo et al, 2017, Ann Transl Med, 5, 159). Almost all combinations failed to show any benefit. Indeed, only one combination with tenofovir achieves higher rates of HBsAg (hepatitis B surface Antigen) loss with a percentage of less than 10 %. However, such a low rate of HBsAg loss makes cure remaining elusive.

However, there is always a need for better therapies to meet the treatment goals in HBV infection, in particular CHB infection.

### SUMMARY OF THE INVENTION

The inventors surprisingly identified that EYP001 has a synergistic effect with interferon for the treatment of hepatitis B, especially on pre-genomic viral RNA, a marker of viral replication, and on HBcrAg, a core related antigen which is a serum marker of chronic hepatitis B. Accordingly, EYP001 and IFN have a synergistic effect on the reduction of cccDNA transcription. These effects were observed after only 4 weeks of treatment which is a very short period of time, whereas neither EYP001 and nor IFN when used alone do not show a significant effect at the same dose and after the same period of time. In addition, surprisingly, the synergistic effect is at least two-fold stronger when EYP001 is administered once a day in comparison to an administration twice a day with the same daily dosing.

Therefore, the present invention relates to the synergistic combination of EYP001 and IFN for use in the treatment of hepatitis B infection, especially chronic hepatitis B. The scope of the invention is defined by the appended claims. The references to the methods of treatment by therapy of this description are to be interpreted as references to compounds, pharmaceutical compositions and medicaments of the present invention for use in those methods.

The present invention relates to EYP001 or a pharmaceutic composition comprising it for use in combination with interferon alpha (IFN-α) in pegylated form for the treatment of hepatitis B virus infection, especially chronic hepatitis B, wherein EYP001 and pegylated IFN-α are used so as to obtain a synergistic effect for decreasing the HBV replication.

It also relates to the use of EYP001 or a pharmaceutical composition comprising it for the manufacture of a drug for the treatment of hepatitis B virus infection, especially chronic hepatitis B, in combination with interferon alpha (IFN-α) in pegylated form wherein EYP001 and IFN-α are used so as to obtain a synergistic effect for decreasing the HBV replication. It further relates to a method for treating a hepatitis B virus infection, especially chronic hepatitis B, in a subject in need thereof, comprising administering a therapeutically effective or sub-therapeutic amount of EYP001 and administering a therapeutically effective or sub-therapeutic amount of interferon alpha (IFN-α) in pegylated form wherein EYP001 and IFN-α are administered so as to obtain a synergistic effect for decreasing the HBV replication.

In one aspect, EYP001 is to be administered at a dose in the range from 50 to 800 mg per day. More particularly, EYP001 is to be administered at a dose in the range from 100 to 600 mg, preferably from 200 to 400 mg per day. Optionally, EYP001 is to be administered at a sub-therapeutic amount.

In one aspect, EYP001 is to be administered once a day. In another aspect, EYP001 is to be administered twice a day.

In one aspect, the pegylated IFN-α is IFN-α2a, IFN-α2b Preferably, IFN-α in pegylated form is to be administered by subcutaneous route once a week. Optionally, IFN-α in pegylated form can be administered at a sub-therapeutic amount.

In one particular aspect, both EYP001 and IFN-α in pegylated form are to be administered at sub-therapeutic amounts.

In one aspect, EYP001 and IFN-α in pegylated form are to be administered during a period of time from 5 6, 7 or 8 weeks to 52 weeks.

In one aspect, EYP001 and IFN-α in pegylated form are to be used in combination with at least one additional active ingredient. More specifically, the at least one additional active ingredient is a polymerase inhibitor selected from the group consisting of L-nucleosides, deoxyguanosine analogs and nucleoside phosphonates. In a very specific aspect, the at least one additional active ingredient is selected from the group consisting of lamivudine, telbivudine, emtricitabine, entecavir, adefovir and tenofovir.

### BRIEF DESCRIPTION OF THE FIGURES

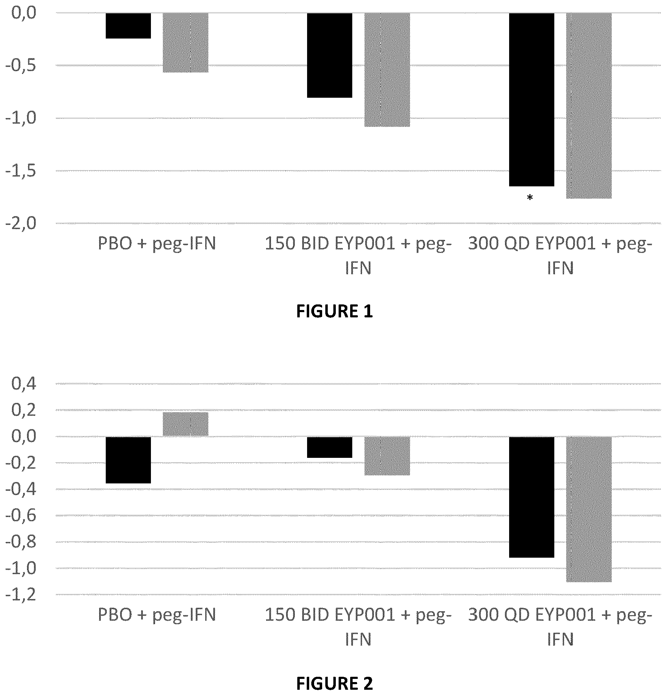

**Figure 1****: HBV pgRNA (log10 copies/mL) changes from baseline after a 4 week anti-HBV treatment course of FXR agonist EYP001a or placebo in combination with interferon in chronically infected previously untreated HBV patients.** *PBO: placebo. peg-IFN: pegylated interferon alpha2a. 150 BID: 150mg twice daily. 300 QD: 300mg once daily. pgRNA: pregenomic ribonucleic acid. Black columns are changes at end of treatment Day 29. Grey columns are changes one week after end of treatment of Day* 35. *^{∗}p=0.04*
**Figure 2****: HBcrAg (log10 IU/mL) changes from baseline after a 4 week anti-HBV treatment course of FXR agonist EYP001a or placebo in combination with interferon in chronically infected previously untreated HBV patients.** *PBO: placebo. peg-IFN: pegylated interferon alpha2a. HBcrAg: hepatitis B core-related antigen. Black columns are changes of end of treatment Day 29. Grey columns are changes one week after end of treatment of Day 35.*

### DETAILED DESCRIPTION OF THE INVENTION

EYP001 is an FXR agonist disclosed in CAS number 1192171-69-9 and having the following formula:

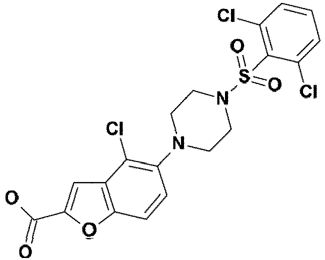

By EYP001 is intended to refer to this compound and any pharmaceutically acceptable salt thereof.

The inventors observed that a combined treatment of EYP001 with pegylated IFN-α surprisingly leads to a synergistic effect on chronic hepatitis B after only 4 weeks of treatment, a very short period whereas none of EYP001 or IFN-α alone shows any significant effect. The effect has been observed on the pre-genomic RNA (HBV pgRNA) and on the hepatitis B core related antigen (HBcrAg). Therefore, a therapeutic benefit can be obtained for the patient by using the synergistic combination of EYP001 with pegylated IFN-α.

### Definition

As used herein, the terms "treatment", "treat" or "treating" refer to any act intended to ameliorate the health status of patients such as therapy, prevention, prophylaxis and retardation of a disease. In certain embodiments, such terms refer to the amelioration or eradication of the disease, or symptoms associated with it. In other embodiments, this term refers to minimizing the spread or worsening of the disease, resulting from the administration of one or more therapeutic agents to a subject with such a disease. More particularly, the term "treating", or "treatment", means alleviating HBV infection, arresting disease development, and/or removing HBV by administering the composition.

By decreasing HBV replication, it is preferably meant that the HBV replication is decreased by at least 10 or 100 fold in comparison with the HBV replication in absence of

More particularly, the treatment of hepatitis B infection, especially chronic hepatitis B, is shown by a decrease of HBV replication. The HBV replication can be assessed by determining at least one of HBeAg levels, HBsAg levels, HBcrAg levels, pre-genomic RNA (HBV pgRNA) levels, pre-core RNA levels, relaxed circular DNA (HBV rcDNA) levels, HBV cccDNA levels or HBV DNA levels in the subject. HBsAg loss and seroconversion are generally the goal for clinical cure. By decreasing, it is meant that the level in at least one of HBeAg levels, HBsAg levels, HBcrAg levels, pre-genomic RNA (HBV pgRNA) levels, pre-core RNA levels, relaxed circular DNA (HBV rcDNA) levels, HBV cccDNA levels and HBV DNA levels is decreased in comparison with the absence of treatment.

By decreasing HBV replication, it is preferably meant that the HBV replication is decreased by at least 10 or 100 fold in comparison with the HBV replication in absence of treatment. For instance, the HBV replication can be assessed by determining the HBV DNA levels and this level is decreased by at least 10 or 100 fold in comparison with the HBV replication in absence of EYP001. Alternatively, HBV cccDNA level is decreased by at least 10, 15, 20, 25, 30, 35, 40, 45 or 50 % in comparison with the absence of treatment.

As used herein, the terms "subject", "individual" or "patient" are interchangeable and refer to a human, including adult, child, newborn and human at the prenatal stage. In a particular aspect, the subject or patient suffers of hepatitis B infection, in particular a chronic hepatitis B.

The terms "quantity," "amount," and "dose" are used interchangeably herein and may refer to an absolute quantification of a molecule.

As used herein, the term "therapeutic effect" refers to an effect induced by an active ingredient, or a pharmaceutical composition according to the invention, capable to prevent or to delay the appearance or development of a disease or disorder, or to cure or to attenuate the effects of a disease or disorder.

As used herein, the term "therapeutically effective amount" refers to a quantity of an active ingredient or of a pharmaceutical composition which prevents, removes or reduces the deleterious effects of the disease, particularly infectious disease. It is obvious that the quantity to be administered can be adapted by the man skilled in the art according to the subject to be treated, to the nature of the disease, etc. In particular, doses and regimen of administration may be function of the nature, of the stage and of the severity of the disease to be treated, as well as of the weight, the age and the global health of the subject to be treated, as well as of the judgment of the doctor.

As used herein, the term "sub-therapeutic amount" or "sub-therapeutic dose" refers to a dosage which is less than that dosage which would produce a therapeutic result in the subject if administered in the absence of the other agent. For instance, "sub-therapeutic amount" or "sub-therapeutic dose" can refer to a dosage which is decreased by 25, 50, 70, 80 or 90 % in comparison to the therapeutically effective amount, especially the conventional therapeutic dosage for the same indication and the same administration route when used alone. The conventional therapeutic dosages are those acknowledged by the drug approvals agencies (e.g., FDA or EMEA).

As used herein, the term "excipient or pharmaceutically acceptable carrier" refers to any ingredient except active ingredients that is present in a pharmaceutical composition. Its addition may be aimed to confer a particular consistency or other physical or gustative properties to the final product. An excipient or pharmaceutically acceptable carrier must be devoid of any interaction, in particular chemical, with the active ingredients.

As used herein, the term "pegylated form" refers to a pegylated interferon.

By "a synergistic effect" is intended to refer to an effect for decreasing the HBV replication which is more than the sum of the effects of each molecule alone. HBV replication can be assessed by determining surface HBV antigen (HBsAg), HBeAg, HBV core related antigen (HBcrAg), HBV DNA, HBV pre-genomic RNA, HBV pre-core RNA and/or HBV cccDNA. More particularly, the effect is observed on the pre-genomic RNA (HBV pgRNA) and/or on the hepatitis B core related antigen (HBcrAg).

### Combined treatment

The present invention relates to the use of a combination of EYP001 and IFN for the treatment of hepatitis B virus infection, especially chronic hepatitis B. Indeed, this combination leads to a synergistic effect against HBV.

Accordingly, the present invention relates to
- a pharmaceutical composition comprising EYP001 and IFN-α in pegylated form and optionally a pharmaceutically acceptable carrier and/or an additional active ingredient, in particular for use in the treatment of hepatitis B virus infection, especially chronic hepatitis B, wherein EYP001 and pegylated IFN-α are used so as to obtain a synergistic effect for decreasing the HBV replication;
- a product or kit containing EYP001 or a pharmaceutical composition comprising it and IFN-α in pegylated form as a combined preparation for simultaneous, separate or sequential use, in particular in the treatment of hepatitis B virus infection, especially chronic hepatitis B, wherein EYP001 and IFN-α are used so as to obtain a synergistic effect for decreasing the HBV replication; optionally, the product or kit may comprise at least one additional active ingredient;
- a combined preparation which comprises EYP001 or a pharmaceutical composition comprising it and IFN-α in pegylated form for simultaneous, separate or sequential use, in particular in the treatment of hepatitis B virus infection, especially chronic hepatitis B, wherein EYP001 and IFN-α are used so as to obtain a synergistic effect for decreasing the HBV replication; optionally, the combined preparation may comprise at least one additional active ingredient;
- a pharmaceutical composition comprising EYP001 for the use in the treatment of hepatitis B virus infection, especially chronic hepatitis B, in combination with a treatment with IFN-α in pegylated form wherein EYP001 and IFN-α are used so as to obtain a synergistic effect for decreasing the HBV replication; optionally, the pharmaceutical composition may comprise at least one additional active ingredient;
- a pharmaceutical composition comprising IFN-α or a pegylated form thereof for the use in the treatment of hepatitis B virus infection, especially chronic hepatitis B, in combination with a treatment with EYP001, wherein EYP001 and IFN-α are used so as to obtain a synergistic effect for decreasing the HBV replication; optionally, the pharmaceutical composition may comprise at least one additional active ingredient;
- the use of a pharmaceutical composition comprising EYP001 for the manufacture of a medicament for the treatment of hepatitis B virus infection, especially chronic hepatitis B, in combination with a treatment with IFN-α in pegylated form wherein EYP001 and IFN-α are used so as to obtain a synergistic effect for decreasing the HBV replication; optionally, the pharmaceutical composition may comprise at least one additional active ingredient;
- the use of a pharmaceutical composition comprising IFN-α in pegylated form for the manufacture of a medicament for the treatment of hepatitis B virus infection, especially chronic hepatitis B, in combination with a treatment with EYP001, wherein EYP001 and IFN-α are used so as to obtain a synergistic effect for decreasing the HBV replication; optionally, the pharmaceutical composition may comprise at least one additional active ingredient;
- the use of a pharmaceutical composition comprising EYP001 and IFN-α in pegylated form and optionally a pharmaceutically acceptable carrier for the manufacture of a medicament for the treatment of hepatitis B virus infection, especially chronic hepatitis B, wherein EYP001 and IFN-α are used so as to obtain a synergistic effect for decreasing the HBV replication; optionally, the pharmaceutical composition may comprise at least one additional active ingredient;

The IFN-α can be for instance IFN-α1 or IFN-α2, e.g., IFN-α1α, IFN-α1b, IFN-α2a, IFN-α2b, IFN-α2c or consensus IFN-α. In a very particular aspect, IFN is IFN-α2a, IFN-α2b or a pegylated form thereof.

Optionally, IFN-α is selected from the non-exhaustive list consisting of consensus IFN-α (e.g., INFERGEN^{®}, Locteron^{®}), IFN-α1b (e.g., HAPGEN^{®}), IFN-α2a (Roferon-A^{®}, MOR-22, Inter 2A, Inmutag, Inferon), a pegylated IFN-α2a (e.g., PEGASYS^{®}, YPEG-IFNα-2a, PEG-INTRON^{®}, Pegaferon), IFN-α2b (e.g., INTRON A^{®}, Alfarona, Bioferon, Inter 2B, citpheron, Zavinex, Ganapar, etc...), a pegylated IFN-α2b (e.g., Pegintron^{®}, Albuferon, AOP2014/P1101, Algeron, Pai Ge Bin), and IFN-α2c (e.g. Berofor Alpha). In a particular aspect, IFN is a pegylated IFN-α2a (e.g., PEGASYS^{®}) or a pegylated IFN-α2b (Pegintron^{®}).

In an aspect, the IFNα in pegylated form is administered by subcutaneous route once a week; for instance, at a dosage varying from 1 µg to 500 µg, preferably from 10 µg to 500 µg, still more preferably from 100 µg to 250 µg, such as 100, 110, 120, 130, 140, 150, 160, 170, 180, 190 or 200 µg.

Optionally, the IFNα in pegylated form can be administered at a sub-therapeutic amount. EYP001 can be administered at a therapeutic amount effective once or twice a day; more specifically, at a daily dose from 50 to 800 mg per adult per day, preferably from 100 to 600 mg per adult per day; still more preferably from 150 to 400 mg per adult per day or from 200 to 400 mg per day and for instance about 300 mg per adult per day; preferably orally.

In the context of a combined treatment with EYP001 and IFN-α (i.e., IFN-α2a, IFN-α2b in pegylated form), the inventors surprisingly observed a synergistic effect is at least twice stronger when EYP001 is administered once a day in comparison with an administration twice a day with the same daily dosing. In addition, the inventors observed that, surprisingly, fewer pruritus occurs when EYP001 is administered once a day rather than twice a day. Therefore, in a particular aspect, EYP001 is administered once a day. In a particular aspect, EYP001 is administered in the evening (e.g., 6 and 10 pm). In an alternative aspect, EYP001 is administered in the morning (e.g., between 6 and 10 am).

Optionally, EYP001 is administered with or without food. Then, in a first aspect, EYP001 is administered during the meal, e.g., just before, simultaneously or just after the meal. In a second aspect, EYP001 is administered at least one hour or two hours before or after the meal.

Preferably, the composition, dosage unit or dosage form contains 5, 10, 15, 25, 50, 75, 100, 200, 300, 400 and 500 mg of EYP001 for the symptomatic adjustment of the dosage to the patient to be treated. A medicament typically contains from about 5 mg to about 500 mg of EYP001, preferably from 50 mg to about 500 mg of EYP001, from 50 mg to about 450 mg of EYP001, from 100 mg to about 400 mg of EYP001, or from 150 mg to about 300 mg of EYP001.

In one aspect, the dosage form can be a scored dosage form. Alternatively, the daily dosage can be provided by administering several dosage forms.

The EYP001 may be combined with pharmaceutically acceptable excipients, and optionally sustained-release matrices, such as biodegradable polymers, to form pharmaceutical compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a nontoxic solid, semi-solid or liquid filler, diluent, encapsulating material or formulation auxiliary of any type. The pharmaceutical compositions comprising EYP001 can be suitable for oral, sublingual, subcutaneous, intramuscular, intravenous, transdermal, local or rectal administration, preferably for oral administration. The EYP001, alone or in combination with another active principle, can be administered in a unit administration form, as a mixture with conventional pharmaceutical supports. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

In a preferred embodiment, the oral dosage form is a capsule or a tablet. Optionally, the oral dosage form is a scored dosage form. Optionally, the dosage form can be scored into four pieces, three pieces or two pieces.

Optionally, the treatment lasts from 2-4 months up to 24 months, for instance between 2 and 24 months or between 2 and 12 months, e.g., 2, 3, 4, 5, 6, 7, 8, 9, 10, 11, 12, 13, 14, 15, 16, 17, 18, 19, 20, 21, 22, 23 or 24 months. In a very specific aspect, the treatment lasts from 12 to 52 weeks, preferably from 45 to 52 weeks, for instance 48 weeks.

EYP001 and IFN-α in pegylated form can be used in combination with at least one additional active ingredient. Preferably, the additional active ingredient is an antiviral, more particularly an antiviral having an activity against HBV. In a preferred aspect, the at least one additional active ingredient is a polymerase inhibitor selected from the group consisting of L-nucleosides, deoxyguanosine analogs and nucleoside phosphonates. In a very specific aspect, the at least one additional active ingredient is selected from the group consisting of lamivudine, telbivudine, emtricitabine, entecavir, adefovir and tenofovir.

Further aspects and advantages of the present invention will be described in the following examples, which should be regarded as illustrative and not limiting.

### EXAMPLES

HBV chronically infected patients (male (n=39) and female (n=34)) underwent a 4-week treatment course as either daily oral FXR agonist EYP001a monotherapy or placebo or entecavir (ETV) in Part A of the study (n=48) or in combination with interferon (n=23, weekly sub-cutaneous injections of pegylated IFNα2a, PEG-IFN) in Part B. Patient characteristics were: mean age of 39.7 years (range: 19- 63); 6 of 73 were HBeAg-positive; 70% treatment naïve; mean baselines HBV DNA 4.2 (±1.5 SD) log10 IU/mL, HBsAg 3.5 (±0.8 SD) log10 IU/mL, and genotypes A (25), B(8), C(10), D(7) and E(4). Detailed virology characteristics are summarized in Table 2 and 3. FXR engagement with all EYP001 doses led to decreases in C4 and increases in FGF19 (data not shown).

At end of treatment on day 29, 400mg QD EYP001 decrease the mean HBsAg by -0.1 loglO IU/mL (p<0.05). Surprisingly early markers of HBV replication pgRNA and HBcrAg showed a synergistic decrease when EYP001 was combined with peg-IFN, but not with peg-IFN or EYP001 monotherapies (Table 1). The mean HBV pgRNA decrease was -1.7 log10 Copies/mL (p<0.05) and the mean HBcrAg decrease was -0.9 log10 IU/mL (p=0.15), whereas the Placebo + Peg-IFN group had no significant decline (-0.2 log10 Copies/mL pgRNA, -0.4 log10 IU/mL HBcrAg). This effect lasted at day 35, i.e. 7 days after end of treatment (EoT, Fig 1 and Fig 2). A stronger synergistic effect is observed with QD in comparison to BID.

**Table 1**

| | **HBVpgRNA Log10 Copies mL** | | | | **HBcrAg Log10 IU mL** | | |
|---|---|---|---|---|---|---|---|
| **Treatment** | **n** | **Mean** | **SD** | **P-value (a)** | **Mean** | **SD** | **P-value (a)** |
| **ETV (open-label 0.5 mg/day)** | 7 | -0.3 | 2.0 | 0.67 | 0.03 | 0.6 | 0.91 |
| **EYP001a (1x100 mg/day)** | 7 | 0.5 | 1.2 | 0.33 | -0.3 | 1.1 | 0.45 |
| **EYP001a (1x200 mg/day)** | 8 | 0.4 | 1.7 | 0.59 | 0.2 | 1.1 | 0.56 |
| **EYP001a (1x400 mg/day)** | 8 | 0.2 | 1.4 | 0.65 | 0.02 | 0.1 | 0.50 |
| **EYP001a (2x200 mg/day)** | 7 | -0.1 | 1.9 | 0.92 | 1.2 | 2.2 | 0.19 |
| **EYP001a (1x300 mg/day) +Peg-IFN** | 8 | **-1.7** | **1.9** | **0.04 *** | **-0.9** | **1.6** | **0.15** |
| **EYP001a (2x150 mg/day) +Peg-IFN** | 8 | **-0.9** | **1.8** | **0.19** | **-0.2** | **0.2** | **0.06** |
| **Placebo +Peg-IFN** | 8 | -0.2 | 1.0 | 0.52 | -0.4 | 0.9 | 0.30 |
| **(*) From Student paired test.** | | | | | | | |

**Table 2: Summary of HBV infection parameters at baseline for Part A**

| | **EYP001a 1x100mg (N=7)** | **EYP001a 1x200 mg (N=8)** | **EYP001a 1x400 mg (N=9)** | **EYP001a 2x200 mg (N=9)** | **EYP001a Total (N=33)** | **ETV 0.5 (mg/day) (N=7)** | **All Subjects (N=48)** |
|---|---|---|---|---|---|---|---|
| ALT (measn, SD) | 36.2 (20.8) | 28.6(11.0) | 22.3 (6.1) | 32.4 (12.5) | **29.7 (13.5)** | 24.0 (10.9) | 28.4 (12.4) |
| Treatment naive | 5 (71%) | 6 (75%) | 8 (89%) | 8 (89%) | **27 (82%)** | 4 (57%) | 35 (73%) |
| HBV DNA baseline mean log10 IU/mL (SD) | 4.76 (1.15) | 3.46 (0.44) | 3.84 (0.77) | 4.35 (1.9) | **4.08 (1.26)** | 3.82 (0.70) | 4.03 (1.20) |
| HBsAg Igo10 IU/mL (SD) | 3.4 (0.54) | 3.5 (0.6) | 3.7 (0.6) | 3.3 (1.0 | **3.5 (0.7)** | 3.2 (0.6) | 3.5 (0.7) |
| HBV Genotype A | 2 (29%) | 2 (25%) | 2 (22%) | 3 (33%) | **9 (27%)** | 3 (43%) | 12 (25%) |
| HBV Genotype B or C or D or E | 3 (xx%) | 5 (xx%) | 5 (%) | 3 (%) | **16 (%)** | 1 (14%) | 20 (%) |
| HBeAg neg | 6 (86%) | 7 (88%) | 9 (100%) | 7 (78%) | **29 (88%)** | 7 (100%) | 42 (88%) |

**Table 3: Summary of HBV infection parameters at baseline for Part B**

| | **EYP001a (1x300 mg) +PEG-IFN(18µg) (N=8)** | **EYP001a (2x150 mg) +PEG-IFN(18µg) (N=9)** | **EYP001a + PEG-IFN Total (N=17)** | **Placebo + PEG-IFN(18µg) (N=8)** | **All Subjects (N=25)** |
|---|---|---|---|---|---|
| ALT (mean, SD) | 34.7 (38.6) | 27.3 (13.5) | **30.4 (26.2)** | 32.2 (12.4) | 31.0 (22.6) |
| HBV DNA baseline mean log10 IU/mL (SD) | 4.17 (1.79) | 4.26 (1.80) | **4.22 (1.74)** | 4.87 (2.49) | 4.42 (1.98) |
| HBsAg Igo10 IU/mL (SD) | 3.8 (0.6) | 3.4 (1.1) | **3.6 (0.9)** | 3.9 (0.8) | 3.7 (0.9) |
| HBV Treatment naive | 5 (63%) | 8 (89%) | 13 (76%) | 3 (38%) | 16 (64%) |
| HBV Genotype A | 3 (38%) | 1 (11%) | 4 (24%) | 4 (50%) | 8 (32%) |
| HBV Genotype B | 1 (13%) | - | 1 (6%) | 2 (25%) | 3 (12%) |
| HBV Genotype C | 1 (13%) | 2 (22%) | 3 (18%) | - | 3 (12%) |
| HBV Genotype D | - | 1 (11%) | 1 (6%) | 1 (13%) | 2 (8%) |
| HBV Genotype E | 1 (13%) | - | 1 (6%) | - | 1 (4%) |
| HBeAg neg | 7 (88%) | 7 (78%) | 14 (82%) | 5 (63%) | 19 (76%) |
| HBeAg pos | | 1 (11%) | 1 (6%) | 1 (13%) | 2 (8%) |
| anti-HBeAg pos | 7 (88%) | 7 (78%) | 14 (82%) | 5 (63%) | 19 (76%) |

## Claims

1. EYP001 for use in combination with a pegylated interferon alpha (IFN-α) for the treatment of hepatitis B virus infection, wherein EYP001 and IFN-α are used so as to obtain a synergistic effect for decreasing the HBV replication.

2. EYP001 for use according to claim 1, wherein EYP001 is to be administered at a dose in the range from 50 to 800 mg per day.

3. EYP001 for use according to claim 1, wherein EYP001 is to be administered at a dose in the range from 100 to 600 mg per day, preferably from 200 to 400 mg per day.

4. EYP001 for use according to any one of claims 1 to 3, wherein EYP001 is to be administered once a day.

5. EYP001 for use according to any one of claims 1 to 3, wherein EYP001 is to be administered twice a day.

6. EYP001 for use according to any one of claims 1-5, wherein the pegylated IFN-α is a pegylated IFN-α2a and a pegylated IFN-α2b.

7. EYP001 for use according to any one of claims 1-6, wherein the pegylated IFN-α is to be administered at a sub-therapeutic amount.

8. EYP001 for use according to any one of claims 1-7, wherein the pegylated IFN-α is to be administered by subcutaneous route once a week.

9. EYP001 for use according to any one of claims 1 and 6-8, wherein EYP001 is to be administered at a sub-therapeutic amount.

10. EYP001 for use according to any one of claims 1-9, for use for the treatment of chronic hepatitis B infection.

11. EYP001 for use according to any one of claims 1-10, wherein EYP001 and the pegylated IFN-α are to be administered during a period of time from 5, 6, 7 or 8 weeks to 52 weeks.

12. EYP001 for use according to any one of claims 1-11, wherein EYP001 and the pegylated IFN-α are to be used in combination with at least one additional active ingredient.

13. EYP001 for use according to claim 12, wherein the at least one additional active ingredient is a polymerase inhibitor selected from the group consisting of L-nucleosides, deoxyguanosine analogs and nucleoside phosphonates.

14. EYP001 for use according to claim 12, wherein the at least one additional active ingredient is selected from the group consisting of lamivudine, telbivudine, emtricitabine, entecavir, adefovir and tenofovir.

## Patentansprüche

1. EYP001 zur Verwendung in Kombination mit einem pegylierten Interferon alpha (IFN-α) zur Behandlung einer Hepatitis-B-Virusinfektion, wobei EYP001 und IFN-α so verwendet werden, dass ein synergistischer Effekt zur Verringerung der HBV-Replikation erzielt wird.

2. EYP001 zur Verwendung nach Anspruch 1, wobei EYP001 in einer Dosis im Bereich von 50 bis 800 mg pro Tag zu verabreichen ist.

3. EYP001 zur Verwendung nach Anspruch 1, wobei EYP001 in einer Dosis im Bereich von 100 bis 600 mg pro Tag zu verabreichen ist, vorzugsweise von 200 bis 400 mg pro Tag.

4. EYP001 zur Verwendung nach einem der Ansprüche 1 bis 3, wobei EYP001 einmal am Tag zu verabreichen ist.

5. EYP001 zur Verwendung nach einem der Ansprüche 1 bis 3, wobei EYP001 zweimal am Tag zu verabreichen ist.

6. EYP001 zur Verwendung nach einem der Ansprüche 1 bis 5, wobei das pegylierte IFN-α ein pegyliertes IFN-α2a und ein pegyliertes IFN-α2b ist.

7. EYP001 zur Verwendung nach einem der Ansprüche 1 bis 6, wobei das pegylierte IFN-α in einer subtherapeutischen Menge zu verabreichen ist.

8. EYP001 zur Verwendung nach einem der Ansprüche 1 bis 7, wobei das pegylierte IFN-α einmal in der Woche auf einem subkutanen Weg zu verabreichen ist.

9. EYP001 zur Verwendung nach einem der Ansprüche 1 und 6 bis 8, wobei das EYP001 in einer subtherapeutischen Menge zu verabreichen ist.

10. EYP001 zur Verwendung nach einem der Ansprüche 1 bis 9, zur Verwendung bei der Behandlung einer chronischen Hepatitis-B-Infektion.

11. EYP001 zur Verwendung nach einem der Ansprüche 1 bis 10, wobei EYP001 und das pegylierte IFN-α während einer Dauer von 5, 6, 7 oder 8 Wochen bis 52 Wochen zu verabreichen ist.

12. EYP001 zur Verwendung nach einem der Ansprüche 1 bis 11, wobei EYP001 und das pegylierte IFN-α in Kombination mit mindestens einem zusätzlichen aktiven Inhaltsstoff zu verwenden sind.

13. EYP001 zur Verwendung nach Anspruch 12, wobei der mindestens eine zusätzliche aktive Inhaltsstoff ein Polymeraseinhibitor ausgewählt aus der Gruppe bestehend aus L-Nukleosiden, Desoxyguanosin-Analoga und Nukleosidphosphonaten ist.

14. EYP001 zur Verwendung nach Anspruch 12, wobei der mindestens eine zusätzliche aktive Inhaltsstoff ausgewählt ist aus der Gruppe bestehend aus Lamivudin, Telbivudin, Emtricitabin, Entecavir, Adefovir und Tenofovir.

## Revendications

1. EYP001 pour une utilisation en combinaison avec un interféron alpha (IFN-α) PEG-ylé pour le traitement d'une infection par le virus de l'hépatite B, dans lequel l'EYP001 et l'IFN-α sont utilisés de façon que soit obtenu un effet synergique pour réduire la réplication de VHB.

2. EYP001 pour une utilisation selon la revendication 1, lequel EYP001 est destiné à être administré à une dose située dans la plage allant de 50 à 800 mg par jour.

3. EYP001 pour une utilisation selon la revendication 1, lequel EYP001 est destiné à être administré à une dose située dans la plage allant de 100 à 600 mg par jour, de préférence de 200 à 400 mg par jour.

4. EYP001 pour une utilisation selon l'une quelconque des revendications 1 à 3, lequel EYP001 est destiné à être administré une fois par jour.

5. EYP001 pour une utilisation selon l'une quelconque des revendications 1 à 3, lequel EYP001 est destiné à être administré deux fois par jour.

6. EYP001 pour une utilisation selon l'une quelconque des revendications 1 à 5, dans lequel l'IFN-α PEG-ylé est un IFN-α2a PEG-ylé et un IFN-α2b PEG-ylé.

7. EYP001 pour une utilisation selon l'une quelconque des revendications 1 à 6, dans lequel l'IFN-α PEG-ylé est destiné à être administré en une quantité sous-thérapeutique.

8. EYP001 pour une utilisation selon l'une quelconque des revendications 1 à 7, dans lequel l'IFN-α PEG-ylé est destiné à être administré par voie sous-cutanée une fois par semaine.

9. EYP001 pour une utilisation selon l'une quelconque des revendications 1 et 6 à 8, lequel EYP001 est destiné à être administré en une quantité sous-thérapeutique.

10. EYP001 pour une utilisation selon l'une quelconque des revendications 1 à 9, pour une utilisation dans le traitement d'une infection hépatite B chronique.

11. EYP001 pour une utilisation selon l'une quelconque des revendications 1 à 10, dans lequel l'EYP001 et l'IFN-α PEG-ylé sont destinés à être administrés pendant une période de 5, 6, 7 ou 8 semaines à 52 semaines.

12. EYP001 pour une utilisation selon l'une quelconque des revendications 1 à 11, dans lequel l'EYP001 et l'IFN-α PEG-ylé sont destinés à être utilisés en combinaison avec au moins un principe actif additionnel.

13. EYP001 pour une utilisation selon la revendication 12, dans lequel l'au moins un principe actif additionnel est un inhibiteur de polymérase choisi dans le groupe constitué par les L-nucléosides, les analogues de désoxyguanosine et les phosphonates de nucléosides.

14. EYP001 pour une utilisation selon la revendication 12, dans lequel l'au moins un principe actif additionnel est choisi dans le groupe constitué par la lamivudine, la telbivudine, l'emtricitabine, l'entécavir, l'adéfovir et le ténofovir.
